# EUROPEAN PATENT APPLICATION

(11) **EP 2 609 910 A1**
(43) Date of publication of application: **03.07.2013**
(21) Application number: 11196237.9
(22) Date of filing: 30.12.2011
(51) Int. Cl.: A61K 9/00

(54) **Formulations of eprotirome**

(71) Applicant: Deva Holding Anonim Sirketi, 34303 Istanbul (TR)
(72) Inventor: Haas, Philipp Daniel, 34303 ISTANBUL (TR); Koc, Fikret, 34303 ISTANBUL (TR); Sivasligil, Ramazan, 34303 ISTANBUL (TR); Kandemir, Levent, 34303 ISTANBUL (TR); Ilhan, Suna Ayse, 34303 ISTANBUL (TR)

(57) **Abstract**

The present invention is related to easy to use dosage forms for low formulations of eprotirome comprising orally disintegrating tablets, effervescent tablets, granules in sachets, dry syrup and manufacturing methods thereof.

## Description

### Technical Field

The present invention is related to easy to use dosage forms for low formulations of eprotirome comprising orally disintegrating tablets, effervescent tablets, granules in sachets, dry syrup and manufacturing methods thereof.

### Background Art

Recently, high cholesterol levels in the blood are primarily treated with a group of drugs called statins, but they are not always sufficiently effective and higher doses commonly cause adverse reactions.

But recent understanding of thyroid hormone receptors has led to the development of thyroid hormone mimetics which are potential therapeutic agents to lower cholesterol. Among them, eprotirome has drawn attention during clinical trials with its promising efficacy and safety profile for the treatment of heterozygous familial hypercholesterolemia (HeFH).

Eprotirome mimics the natural ability of thyroid hormone to stimulate the metabolism of cholesterol, and exerts its effects exclusively on the liver.

It is reported that eprotirome show significant reduction in LDL cholesterol in patients already taking lipid-lowering drugs and lower triglycerides.

Eprotirome is a liver selective thyroid hormone receptor (THR) agonist and indicated for the treatment of dyslipidemia.

Eprotirome is described as agonist of thyroid hormone receptor especially TRß receptor.

Eprotirome is chemically described as N-[3,5-dibromo-4-(4-hydroxy-3-isopropylphenoxy)phenyl] malonamic acid.

Eprotirome is firstly disclosed by WO 01/060784 A (BRISTOL MYERS SQUIBB CO.) 23.08.2001

WO 2007/110226 A (KARO BIO AB) 04.10.2007 describes compositions in which thyroid hormone receptor binding compounds are formulated together with either an enteric coating or an antioxidant or both.

WO 2009/077147 A (KARO BIO AB) 25.06.2009 describes compositions in which thyroid hormone receptor binding compounds are formulated with a basic filler to prevent the formation of undesired reaction products.

As people are living longer, drug dosage forms that can improve patient compliance are needed. Better patient compliant and easy-to-use dosage forms are becoming increasingly popular compared to conventional solid dosage forms which are most commonly used for oral administration.

Patient friendly dosage forms can be conveniently presented in the suitable form of orally disintegrating tablet, sachet, effervescent tablet and dry syrup to enhance the patient compliance and administration convenience in the treatment.

Recent market studies showed that orally disintegrating tablet is preferred by more than half of the patient population due to its ease in administration.

The CDER Nomenclature Standards Committee defined orally disintegrating tablet (ODT) as a solid dosage form containing medicinal substances which disintegrates rapidly, usually within a matter of seconds, when placed upon tongue.

In orally disintegrating dosage forms, pharmaceutical formulations are disintegrated in less than three minutes in oral cavity. More preferably, the pharmaceutical formulations are disintegrated in an oral cavity in less than two minutes and most preferably the pharmaceutical formulations are disintegrated in oral cavity in less than one minute.

Orally disintegrating tablets are especially preferable by patients with esophageal problems, who experience difficulties in swallowing or by paediatric, bedridden, geriatrics, psychiatrics who refuse to swallow.

Due to their convenience to carry and ease to take, travelling patients who may not have access to water also prefer such preparations.

Orally disintegrating tablets are also known as orodispersible, rapimelts, quick dissolving, rapid disintegrating, rapid dissolving, mouth dissolving, melt-in-mouth, mouth dispersing, fast mouth dissolving, fast melting, porous tablet, orodispersing tablets.

Furthermore, other easy-to-use formulations include effervescent tablet, granules in sachets or dry syrup or any other dosage in such forms of eprotirome need to be reconstituted with water or other suitable liquid before use and may be administered as fully dissolved, dispersed or suspended in a solution.

However, few formulations of eprotirome have been described in the past and none of these formulations discloses easy-to-use dosage forms and formulations of eprotirome.

### Summary of invention

The present invention is related to easy to use dosage forms for low formulations of eprotirome comprising orally disintegrating tablets, effervescent tablets, granules in sachets, dry syrup or other drug in solution and manufacturing methods thereof.

### Technical Problem

Difficulty in swallowing which is known as dysphagia is common among all age groups. Person with dysphagia or elderly people or paediatrics experience pain when trying to swallow traditional solid oral dosage forms.

Their non compliance with prescription results in inefficient treatment. Moreover patients who just stop taking their medications without informing their physicians may cause total failure of the treatment.

But still, physicians emphasize the importance of continuous medication in the treatment of dyslipidemia and seek alternative dosage forms for complaining patients.

On the other hand, building up appropriate pharmaceutical formulation and a related production method is a challenge to formulation scientist during product development phase. The problem is even more problematic for a low dose drug where it is difficult to ensure the content uniformity and physical stability.

Assuring the content uniformity represents a major quality assurance consideration during the entire manufacturing of finished product. Non homogeneity of powder mixture may cause manufacturing problems, hinder the processibility, thereby bringing about unnecessary delays in production.

Moreover, segregation of the powder blend needs to be avoided during filing into granules in sachet, effervescent tablet, dry syrup dosage forms.

Drug's bitter or irritating taste which is needed to be masked in the oral cavity is an additional problem arising in oral administration. Although various conventional or patented manufacturing techniques have been developed lately by many companies for orally disintegrating tablet, development of low dose formulation for oral disintegrating tablet remain a changeling area.

Therefore, the development of a new manufacturing method which does not require any special investment is always needed.

The purpose of the present invention is to develop easy-to-use dosage forms for low dose of eprotirome comprising orally disintegrating tablets, effervescent granules, granules in sachets, dry syrup and manufacturing methods thereof by improving content uniformity across presented formulations, thereby increasing patient compliance in the treatment of dyslipidemia.

None of these formulations discloses an orally disintegrating tablet having fast disintegration in the mouth without the need for water.

### Solution to Problem

In order to enhance the patient compliance and convenience of administration in the treatment, patient friendly dosage forms are conveniently presented in the suitable form of oral disintegrating tablet, effervescent tablet, granules in sachet, and dry syrup. Hence they are particularly designed to be administered to patients who have difficulties to swallowing capsules or tablets.

Presented dosage forms and formulations surpass previously presented problems arising in product formulation and administration of low dose of eprotirome.

The present invention proposes oral disintegrating tablet and liquid oral pharmaceutical compositions which are desirable due to their ease in administration. Relating controllable manufacturing methods are also presented for each oral solid dosage forms.

### Description of embodiments

In the present invention, more accessible, patient compliant, easy to use dosage forms of eprotirome including orally disintegrating tablets, granules in sachets, dry syrup are firstly developed by inventors.

In the following description, the formulation comprises an effective amount of eprotirome and/or pharmaceutically acceptable salt or solvate thereof and any reference to the term "eprotirome" is intended to include the pharmaceutically acceptable derivatives thereof.

In the first aspect of the present invention, orally disintegrating tablet dosage form of eprotirome that dissolves or disperses rapidly when in contact with saliva is provided. Oral disintegrating dosage forms include an effective amount of eprotirome and at least one pharmaceutically acceptable excipient or mixture of excipients.

Orally disintegrating tablets are useful for elderly people, children and patients who have difficulties in swallowing tablets. Such peoples are unwilling and/or unable to swallow traditional solid dosage forms. This is especially the case of pharmaceuticals for paediatric or geriatric use. To solve this problem, tablets that disintegrate rapidly in the mouth were developed.

These oral disintegrating tablets or orally dissolving dosage forms are typically tablets and thin films that do not require water for swallowing.

According to this invention, oral disintegrating dosage forms may be, but not limited to, rapidly disintegrating tablets, lingual strips, sublingual tablets, sublingual strips, lyophilized wafers, granulated particles, gums whatsoever.

An orally disintegrating tablet (ODT) of eprotirome may have many numerous shapes, such as dish-like, ellipsoid, rods, granules, blocks, cubes with rounded edges, or any other shape suitable for pharmaceutical administration.

The time of disintegration of ODTs is generally less than one minute and it is desired to provide minimum disintegrating time in oral cavity tablet hardness is very important.

In this point, hardness of orally disintegrating tablet which is known to be the critical parameter, influence product performance.

In the present invention, the tablets are evaluated for hardness and have sufficient hardness to maintain physical characteristics during manufacturing, handling and storage.

According to this invention, eprotirome orally disintegrating tablet hardness is in the range of 20 N to 100 N. Quick disintegration is provided in 30 seconds if tablet hardness is in the range of 20N to 75N.

The compressed tablets have sufficient mechanical strength which allows them to be packed in normal push-through blister packages and does not require special packaging like peel-off blisters.

In the development of orally disintegrating tablet of eprotirome, selection of proper packaging material and system is important for enhanced stability of the finished product.

In the present invention, the compositions of orally disintegrating tablet of eprotirome can be prepared through using pharmaceutical technologies including, but not limited to, direct compression, wet granulation, spray drying, freeze-drying, heat molding, tablet molding, sublimation, consumable thin film manufacture, tablet and the like.

Patient compliance improvizing agents can be from artificial or natural origin or mixtures thereof. Their function may vary such as sweeteners, flavour enhancers, taste masking agents etc.

Eprotirome orally disintegrating tablet comprises; eprotirome or pharmaceutically acceptable salt thereof is in the range of from 0.001 % to 50 %, diluent is in the range of from 0.1 % to 99%, binder is in the range of from 0.1 % to 20 %, taste masking agent is in the range of from 0.01% to 70%, coating agent is in the range of from 0.1 % to 50 %, pore former is in the range of from 0.01 % to 5 %, disintegrant is in the range of from 0.1 % to 60 %, sweetener is in the range of from 0.1 % to 40 %, stabilizing agent is in the range of from 0.1 % to 5 %, glidant is in the range of from 0.1 % to 10 %, lubricant is in the range of from 0.1 % to 10 %, surfactant is in the range of from 0.1 % to 10 %, patient compliance improvizing agents is in the range of from 0.01% to 50% of total weight of pharmaceutical dosage form. In this formulation, it is possible that one or more elements, such as stabilizing agent, may not be used. In such a case, without some elements, inventors do not aim that such formulation is excluded the invention. Fewer multifunctional excipients can be used instead of more excipients.

Eprotirome orally disintegrating tablet preferably comprises; eprotirome or pharmaceutically acceptable salt thereof is in the range of from 0.01 % to 10 %, diluent is in the range of from 10% to 90%, binder is in the range of from 0.1 % to 10 %, taste masking agent is in the range of from 0.1 % to 50%, coating agent is in the range of from 1 % to 20 %, pore former is in the range of from 0.5 % to 3 %, disintegrant is in the range of from 1% to 40 %, sweetener is in the range of from 2 % to 30 %, stabilizing agent is in the range of from 0.5 % to 3 %, glidant is in the range of from 0.2 % to 5 %, lubricant is in the range of from 0.2 % to 5 %, surfactant is in the range of from 1 % to 8 %, patient compliance improvizing agents is in the range of from 0.1 % to 30% of total weight of pharmaceutical dosage form. In this formulation, it is possible that one or more elements, such as stabilizing agent, may not be used. In such a case, without some elements, inventors do not aim that such formulation is excluded the invention. Fewer multifunctional excipients can be used instead of more excipients.

Eprotirome orally disintegrating tablet more preferably comprises; eprotirome or pharmaceutically acceptable salt thereof is in the range of from 0.1 % to 1 %, diluent is in the range of from 20 % to 70%, binder is in the range of from 1 % to 5 %, taste masking agent is in the range of from 0.2% to 20%, coating agent is in the range of from 2 % to 10 %, pore former is in the range of from 1 % to 2 %, disintegrant is in the range of from 5 % to 20 %, sweetener is in the range of from 5 % to 20 %, stabilizing agent is in the range of from 1 % to 2 %, glidant is in the range of from 0.5 % to 1 %, lubricant is in the range of from 0.5 % to 1 %, surfactant is in the range of from 2 % to 5 %, patient compliance improvizing agents is in the range of from 0.2 % to 20% of total weight of pharmaceutical dosage form. In this formulation, it is possible that one or more elements, such as stabilizing agent, may not be used. In such a case, without some elements, inventors do not aim that such formulation is excluded the invention. Fewer multifunctional excipients can be used instead of more excipients.

In the present invention, other easy-to-use dosage forms such as effervescent tablet, granules in sachet and dry syrup and formulation of eprotirome are developed by inventors. These dosage forms need to be reconstituted with water or other suitable liquid before use and may be administered as fully dissolved, dispersed or suspended in a solution. Hence they are particularly designed to be administered to patients who have difficulties to swallowing capsules or tablets.

Oral administration of the powder or tablet in solution or suspension or dispersion provides particularly several advantages over conventional oral solid dosage forms. As the drug product is already in solution at the time it is consumed, reduced localized contact of the drug in the upper gastrointestinal tract leads to less irritation and greater tolerability to patients.

In the second aspect of the present invention, effervescent tablet of eprotirome is provided. Effervescent tablet dosage form includes an effective amount of eprotirome and at least one pharmaceutically acceptable excipient or mixture of excipients.

Although effervescent tablets are generally added to an aqueous medium such as water prior to oral administration, they may be designed to disintegrate in the mouth.

Effervescent tablet are making use of a chemical reaction between a soluble acid and an alkali metal carbonate with the help of water to make CO₂ gas, which can give a fizzy taste in the mouth.

Typically, the acid is organic or mineral acid that is safe and approved for consumption and pharmaceutical and/or nutritional purposes which provides effective and rapid effervescent disintegration upon contact with water and the alkaline effervescent compound.

The acidity for the effervescent reaction can be obtained from three main sources: acids, acid anhydrides, and acid salts.

The food acids are most commonly used since they are generally regarded as safe (GRAS), they occur in nature and are ingestible. Example of food acid includes are, but not limited to, citric acid, tartaric acid, ascorbic acid, fumaric acid, malic acid, adipic and succinic acid, acetyl salicylic acid, nicotinic acid, mixtures thereof and the like. Citric anhydride, succinic anhyride, glutamic anhydride can be used as well.

The acid salt of the composition can be any suitable acid salt or any mixture of suitable salts. Examples of such a suitable acid salts or mixture of suitable salts includes sodium hydrogen phosphate, disodium dihydrogen pyrophospate, acid citrate salts, aminoacid hydrochlorides. Combinations thereof are possible.

In effervescent formulations, alkaline excipients can be used. The alkaline component can be any suitable alkaline effervescent compound those are safe and approved for pharmaceutical and/or nutritional purposes, and typically it is an organic base (e.g., an alkali metal carbonate). It provides an effective and rapid effervescent disintegration upon contact with water and the acid compound. The alkaline effervescing compound may be selected from the group consisting of carbonate salts, bicarbonate salts, and mixtures thereof.

Example of carbonate sources, but not limited to, sodium bicarbonate, sodium carbonate, potassium bicarbonate and potassium carbonate, sodium sesquicarbonate, sodium glycine carbonate, I-lysine carbonate, arginine carbonate and mixtures thereof. Alkaline earth metal carbonates and bicarbonates can be used but alkali metal carbonates and bicarbonates are preferred.

Any conventional effervescent agent for example a mixture of a substance which on dissolution in water forms an acid reacting solution, and a pharmaceutically acceptable carbonate can be used as effervescent agent.

Eprotirome effervescent tablet comprises; eprotirome or pharmaceutically acceptable salt thereof is in the range of from 0.001 % to 50 %, diluent is in the range of from 0.1 % to 70%, binder is in the range of from 0.1 % to 20 %, taste masking agent is in the range of from 0.01% to 70%, coating agent is in the range of from 0.1 % to 50 %, pore former is in the range of from 0.01 % to 5 %, disintegrant is in the range of from 0.1% to 60 %, sweetener is in the range of from 0.1 % to 40 %, stabilizing agent is in the range of from 0.1 % to 5 %, glidant is in the range of from 0.1 % to 10 %, lubricant is in the range of from 0.1 % to 10 %, surfactant is in the range of from 0.1 % to 10 %, patient compliance improvizing agents is in the range of from 0.01% to 50%, acidic and basic effervescent agents are in the range of from 0.1 % to 80%, of total weight of pharmaceutical dosage form. In this formulation, it is possible that one or more elements, such as stabilizing agent, may not be used. In such a case, without some elements, inventors do not aim that such formulation is excluded the invention. Fewer multifunctional excipients can be used instead of more excipients.

Eprotirome effervescent tablet preferably comprises; eprotirome or pharmaceutically acceptable salt thereof is in the range of from 0.01 % to 10 %, diluent is in the range of from 10% to 50%, binder is in the range of from 0.1 % to 10 %, taste masking agent is in the range of from 0.1 % to 50%, coating agent is in the range of from 1 % to 20 %, pore former is in the range of from 0.5 % to 3 %, disintegrant is in the range of from 1% to 40 %, sweetener is in the range of from 2 % to 30 %, stabilizing agent is in the range of from 0.5 % to 3 %, glidant is in the range of from 0.2 % to 5 %, lubricant is in the range of from 0.2 % to 5 %, surfactant is in the range of from 1 % to 8 %, patient compliance improvizing agents is in the range of from 0.1% to 30%, acidic and basic effervescent agents are in the range of from 1 % to 50%, of total weight of pharmaceutical dosage form. In this formulation, it is possible that one or more elements, such as stabilizing agent, may not be used. In such a case, without some elements, inventors do not aim that such formulation is excluded the invention. Fewer multifunctional excipients can be used instead of more excipients.

Eprotirome effervescent tablet more preferably comprises; eprotirome or pharmaceutically acceptable salt thereof is in the range of from 0.1 % to 1 %, diluent is in the range of from 15 % to 30%, binder is in the range of from 1 % to 5 %, taste masking agent is in the range of from 0.2% to 20%, coating agent is in the range of from 2 % to 10 %, pore former is in the range of from 1 % to 2 %, disintegrant is in the range of from 5 % to 20 %, sweetener is in the range of from 5 % to 20 %, stabilizing agent is in the range of from 1 % to 2 %, glidant is in the range of from 0.5 % to 1 %, lubricant is in the range of from 0.5 % to 1 %, surfactant is in the range of from 2 % to 5 %, patient compliance improvizing agents is in the range of from 0.2 % to 20%, acidic and basic effervescent agents are in the range of from 2 % to 20%, of total weight of pharmaceutical dosage form. In this formulation, it is possible that one or more elements, such as stabilizing agent, may not be used. In such a case, without some elements, inventors do not aim that such formulation is excluded the invention. Fewer multifunctional excipients can be used instead of more excipients.

In the third aspect of the present invention, granules in sachet dosage form of eprotirome are provided.

Granules in sachet dosage form include an effective amount of eprotirome and at least one pharmaceutically acceptable excipient or mixture of excipients.

As used, a sachet is a single dose of a pharmaceutical formulation. The formulation is preferably in the form of a particulate material, sphere, pellets, particle or granulate.

Alternatively, powder, granulate, pellets, spheres, particles of sachet may be in effervescent form or mixtures thereof. The sachet can be formulated as a powder, granulate, pellets, spheres, particles or effervescent powder, effervescent granulate, effervescent pellets, effervescent spheres, effervescent particles or mixtures.

Presented powder, granulate, pellets, spheres, particles or their mixtures of sachet may be formulated for reconstitution with water or another suitable liquid or food. Said powder, granulate, pellets, spheres, particles or their mixtures may be ready to use sachet's formulation which can be consumed directly from the packet.

For the purposes of the present invention a "sachet" is referred herein to any suitable single serving pack, a container, a package, an envelope or a bag and the like. Preferably the sachet is sealed and disposable.

The sachet may be made up from any suitable material, such as plastic, metal foil, paper, and combination thereof. The sachet includes a perforated region or a nick in the edge of the sachet for ease of tearing.

The sachet may be launched and sold individually or may be launched or sold in pack of two or more sachets.

Eprotirome sachet comprises; eprotirome or pharmaceutically acceptable salt thereof is in the range of from 0.001 % to 50 %, diluent is in the range of from 1 % to 50%, binder is in the range of from 3 % to 25 %, taste masking agent is in the range of from 1% to 40%, coating agent is in the range of from 0.1 % to 50 %, pore former is in the range of from 0.01 % to 5 %, disintegrant is in the range of from 0.1 % to 60 %, sweetener is in the range of from 0.1 % to 40 %, stabilizing agent is in the range of from 0.1 % to 5 %, glidant is in the range of from 0.1% to 10 %, lubricant is in the range of from 0.1% to 10 %, surfactant is in the range of from 0.1% to 10 %, patient compliance improvizing agents is in the range of from 0.01% to 50% of total weight of pharmaceutical dosage form. In this formulation, it is possible that one or more elements, such as stabilizing agent, may not be used. In such a case, without some elements, inventors do not aim that such formulation is excluded the invention. Fewer multifunctional excipients can be used instead of more excipients.

Eprotirome sachet preferably comprises; eprotirome or pharmaceutically acceptable salt thereof is in the range of from 0.01 % to 10 %, diluent is in the range of from 10% to 40%, binder is in the range of from 5 % to 20 %, taste masking agent is in the range of from 2% to 20%, coating agent is in the range of from 1 % to 20 %, pore former is in the range of from 0.5 % to 3 %, disintegrant is in the range of from 1% to 40 %, sweetener is in the range of from 2 % to 30 %, stabilizing agent is in the range of from 0.5 % to 3 %, glidant is in the range of from 0.2 % to 5 %, lubricant is in the range of from 0.2 % to 5 %, surfactant is in the range of from 1 % to 8 %, patient compliance improvizing agents is in the range of from 0.1 % to 30% of total weight of pharmaceutical dosage form. In this formulation, it is possible that one or more elements, such as stabilizing agent, may not be used. In such a case, without some elements, inventors do not aim that such formulation is excluded the invention. Fewer multifunctional excipients can be used instead of more excipients.

Eprotirome sachet more preferably comprises; eprotirome or pharmaceutically acceptable salt thereof is in the range of from 0.1 % to 1 %, diluent is in the range of from 18 % to 25%, binder is in the range of from 7 % to 15 %, taste masking agent is in the range of from 5% to 15%, coating agent is in the range of from 2 % to 10 %, pore former is in the range of from 1 % to 2 %, disintegrant is in the range of from 5 % to 20 %, sweetener is in the range of from 5 % to 20 %, stabilizing agent is in the range of from 1 % to 2 %, glidant is in the range of from 0.5 % to 1 %, lubricant is in the range of from 0.5 % to 1 %, surfactant is in the range of from 2 % to 5 %, patient compliance improvizing agents is in the range of from 0.2 % to 20% of total weight of pharmaceutical dosage form. In this formulation, it is possible that one or more elements, such as stabilizing agent, may not be used. In such a case, without some elements, inventors do not aim that such formulation is excluded the invention. Fewer multifunctional excipients can be used instead of more excipients.

In the fourth aspect of the present invention, dry syrup dosage form of eprotirome is provided.

A dry syrup preparation means "preparation with water which is dissolved or suspended before use". The powder is provided in a suitable container such as glass or polyethylene bottle to which prior to use prescribed amount of water is added to give dispersion. The desired amount of the dispersion is taken orally by the patient according to his or her need.

Dry syrup formulation comprises dry powder or granules or pellets or mixtures thereof.

In presented easy-to-use dosage forms for low formulations of eprotirome comprising orally disintegrating tablets, effervescent tablets, granules in sachets, dry syrup, taste is need to be masked in the oral cavity. A patient friendly, pleasant taste a mouth feel is achieved by inventors in the presented formulations.

Taste masking aims to prevent the drug substance from interaction with taste buds. Current taste-masking technology includes applying polymer coating, use of flavour enhancer, complexation with ion exchange resin, inclusion complex formation with cyclodextrin.

In the present invention, a taste masking layer may be created to mask bitter taste of eprotirome.

The taste masking layer may comprise one or more coating agents. The taste masking layer can further include additional excipient or excipients such as, but not limited to, pore former.

The taste-masking layer (as described herein) can be applied to the eprotirome particles by any suitable method, for example fluidized bed coating methods.

Taste masking coating solution may comprise; (a)an inorganic acid or mixtures of inorganic acids or;(b) an organic acid or mixtures of organic acids or;(c) mixture of organic acid(s) and inorganic acid(s) or; (d) an organic solvent or mixtures of organic solvents or; (e) mixtures of one or more organic solvents and any alternative (a) to (e) or (f) de-ionized water and any alternative (a) to (e) or (g) de-ionized water and a coating agent or mixtures of coating agents. (h) Optionally and additionally an excipient or mixtures of excipients.

There are number of technologies available which evaluate objectively taste. Taste sensor, taste chip, taste sensing system, electronic sensor, and electronic tongue rank as the most popular applications. Taste of eprotirome may be masked and assessed by mentioned taste sensors.

According to this invention, easy to use dosage forms comprising orally disintegrating tablets, granules in sachets, dry syrup, drug in solution may include one or more pharmaceutically acceptable excipients, carriers, or diluents. In fast disintegrating or fast dissolving formulations, surfactants, diluents, sweeteners, disintegrants, binders, lubricants, glidants, colorants, flavors, mixtures thereof and the like can be used.

In fast dissolving or fast disintegrating dosage forms one or more taste masking excipients such as flavors, sweeteners, acidic amino acids, lipids, and surfactants can further be used.

In case of easy to use dosage forms for low dose eprotirome formulations, sugar-based excipients, super-disintegrating agents, effervescent agents and further suitable excipients can be used.

Patient compliance improvizing agents can be from artificial or natural origin or mixtures thereof. Their function may vary such as sweeteners, flavour enhancers, taste masking agents etc.

The compositions of the present invention can be prepared through using pharmaceutical technologies including, but not limited to, direct compression, wet granulation, spray drying, freeze-drying, lyophilisation, sublimation, heat molding, tablet molding, sublimation, mass extrusion, cotton candy process, consumable thin film manufacture, tablet and the like

Acceptable excipients are, but not limited to, calcium sulfate, starch, mannitol, kaolin, sorbitol, xylitol, sodium chloride, sodium bicarbonate, citric acid, powdered cellulose derivatives, microcrystalline cellulose, pullulan, silicified microcrystalline cellulose, ammonium bicarbonate, carrageenan, carbohydrates such as Pharmaburst™, magnesium carbonate, tribasic calcium phosphate, calcium sulfate, magnesium oxide, poloxamer,gums, hydroxypropyl methylcellulose, gelatin, mixtures thereof, and the like .

Diluents may be, but not limited to, mannitol, sorbitol, xylitol, microcrystalline cellulose, silicified microcrystalline cellulose, hydroxypropyl methylcellulose, hydroxypropyl cellulose , pullulan and fast dissolving carbohydrates such as Pharmaburst™, mixtures thereof and the like.

Glidants may be, but not limited to, silicon dioxide, colloidal silicon dioxide, calcium silicate, magnesium silicate, magnesium trisilicate, talc, starch , mixtures thereof or whatsoever.

Binders are, but not limited to, sodium alginate,cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth,sorbitol, acacia, alginates, carrageenan, xanthan gum, locust bean gum and gum arabic, waxes,polyacrylamide , mixtures thereof, and whatsoever.

Lubricants may be, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof or whatsoever.

Disintegrants are, but not limited to, sodium starch glycolate, sodium carboxymethyl cellulose, calcium carboxymethyl cellulose, croscarmellose sodium, crospovidone, alginic acid, chitosan, methyl cellulose, microcrystalline cellulose, powdered cellulose, lower alkylsubstituted hydroxypropyl cellulose, polacrilin potassium, starch, pregelatinized starch, sodium alginate, mixtures thereof or whatsoever.

Flavors are, but not limited to, cinnamon oil,essence of apple, essence of pear, essence of peach, essence of grape, essence of strawberry, essence of raspberry, essence of cherry,essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit , vanilla, benzaldehyde, aldehyde C-8 , aldehyde C-9, aldehyde C-12 , tolyl aldehyde, mixtures thereof or whatsoever.

Coating agents are, but not limited to, ethyl cellulose, methyl cellulose, hydroxypropyl-methyl cellulose, hydroxyethyl cellulose, hydroxypropyl cellulose,polyvinylalcohol, polyvinyl acetate , cellulose acetate, cellulose acetate phthalate, methylcellulose phthalate, hydroxymethyl cellulose phthalate, hydroxypropylmethylcellulose phthalate, hydroxypropylmethylcellulose succinate, cellulose acetate butyrate, methacrylate copolymers, polymethacrylates, acetyltributyl citrate, erythritol, glyceryl palmitostearate, tributyl citrate, triethyl citrate, glyceryl monostearate, glycerin monostearate, gelatin, hydrogenated soybean oil, glyceryl monostearate,Hydrogenated oil, carboxymethyl cellulose,sodium carboxymethyl cellulose, sodium carboxymethyl starch, crosscarmellose sodium, aminoalkyl methacrylate copolymer E, low-substituted hydroxypropyl ether of cellulose,polyvinyl pyrollidone,polyethylene glycol,cellulose trimellitate,hydroxymethyl cellulose acetate succinate, resins like shellac, methylmethacrylate-methacrylic acid , methacrylic acid-ethyl acrylate,zein, hydroxyethyl-ethylcellulose phthalate, cellulose aceto succinate, polylactic acid,polyglycolic acid, gelatinized starch, ammonium hydroxide,sodium hydroxide, potassium hydroxide, magnesium hydroxide, calcium hydroxide, gums, liposomes, cyclodextrins, chitosan, gliadin,hordein,prolamine,sucrose, sorbitol, glycerin,triethylamine, diethylamine, trimethylamine, methylamine, dimethylamine, isopropylamine, triethanolamine, diethanolamine, disodium monohydrogen phosphate, dipotassium monohydrogen phosphate, mixtures thereof and the like .

Pore formers are, but not limited to, polyethylene glycol, camphor, polyvinylpyrrolidone ,dextrose,mannose, fructose,sucrose, glucodifructose, calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, mixtures thereof and the like.

Organic solvents, used in preparation of solution are, but not limited to, alcohols such as methanol, ethanol, propyl alcohol, isopropyl alcohol, 2-methyl-1-propanol etc. ;ketones such as acetone, methyl ethyl ketone, methyl isobutyl ketone etc. ; esters such as methyl acetate, ethyl acetate, propyl acetate, isopropyl acetate, ethyl formate etc. ; hydrocarbons such as heptane ; halogenated hydrocarbons such as dichloromethane.

Sweeteners are but not limited to, corn syrup,dextrose, invert sugar, fructose, saccharin, aspartame, acesulfame-K, Stevia rebaudiana, sucralose, sorbitol, mannitol, zylitol, mixtures thereof and the like

Other ingredients such as colorants and titanium dioxide can also be used.

In case of tablet is preferred, it may comprise sugar-based excipients, super-disintegrating agents, effervescent agents and further suitable excipients.

The formulations of the present invention can be produced by using mixing-granulating method, extrusion-granulating method, fluidized bed-granulating method, chopping granulating method, spray-granulating method, and crushing granulating method, one-pot processing method, direct compression, dry granulation, melt granulation, melt congealing, extrusion process or other pharmaceutically acceptable manufacturing methods. Direct compression and wet granulation in fluid bed granulator are preferred.

Eprotirome can be in microparticulate form with one or more excipients. Microparticulation can also be used as taste-masking method.

In another aspect, newly developed formulations results proved improved content uniformity.

The following examples are given for the purpose of illustration of the present invention and should not limit the scope of the invention.

### Example1: Orally Disintegrating Tablet Unit Formula of Eprotirome

**Table 1**

| **Ingredients** | **mg/tablet** | **% by weight** |
|---|---|---|
| Eprotirome | 0.20 | 0.20 |
| Glidant | 0.50 | 0.50 |
| Diluent | 71.33 | 71.33 |
| Colourant | 0.20 | 0.20 |
| Coating Agent | 1.78 | 1.78 |
| Binder | 4.57 | 4.57 |
| Sweetener | 1.03 | 1.03 |
| Disintegrant | 17.14 | 17.14 |
| Flavour | 1.03 | 1.03 |
| Adsorban/Glidant | 1.07 | 1.07 |
| Lubricant | 1.15 | 1.15 |
| **Total** | **100.00** | **100.00** |

### Example2: Orally Disintegrating Tablet Unit Formula of Eprotirome

This invention also provides a preparation method (Figure 1) which has following steps :
1. Eprotirome is mixed with a portion diluent(s)/filler(s).
2. Coating agent is dissolved in a suitable solvent.
3. The powder mixture obtained from step (1) is coated with coating solution prepared in step (1).
4. The granules obtained from (3) are dried and granules obtained from step (3) are sieved with a suitable sieve.
5. Remaining portion of diluent, coating agent and disintegrant are added to the granules obtained from step (4).
6. Sweetener and flavour are added to mixture obtained in step(5),
7. A part of powder mixture obtained from (6) is taken and remaining glidant is added to said powder mixture, mixed and sieved together
8. Obtained mixture from step (7) is added to mixture in step (6) and mixed together.
9. Sieved lubricant is added to the mixture obtained in step(8)
10. Final mixture is tabletted with hardness range 20-75 N. Average tablet is preferably about 45 N

### Example3: Effervescent Tablet Unit Formula of Eprotirome

**Table 2**

| **Ingredients** | **mg/tablet** | **% (w/w)** |
|---|---|---|
| Eprotirome | 0.2 | 0.05 |
| Potassium Bicarbonate | 98.80 | 24.70 |
| Citric Acid Anhydrous | 140.00 | 35.00 |
| Sucralose | 35.00 | 8.75 |
| Sodium Saccharin | 30.00 | 7.50 |
| Maltodextrin | 51.00 | 12.75 |
| Orange Flavour | **30.00** | 7.50 |
| Sodium Stearyl Fumarate | **15.00** | 3.75 |
| **Total Tablet Weight** | **400.00** | **100.00** |

### Example 4: Manufacturing Process of Example 3

Pharmaceutical formulation may be prepared using methods as below without limiting scope of this invention;

1. Eprotirome, sweetener (sucralose and sodium saccharin) are dispersed in a suitable solvent.

2. Diluent is granulated with solution in step (1) using fluid bed granulator.

3. Drying is continued until loss on drying is less than 1.5% (w/w).

4. Basic effervescent agent (potassium bicarbonate) and acidic effervescent agent (citric acid anhydrous) are added to powder mixture in step (3) and mixed.

5. Orange flavour is mixed with powder mixture prepared at step (4).

6. Optionally, powder mixture in step (5) is sieved through suitable sized sieve.

7. Lubricant (sodium stearyl fumarate) is added and mixed to powder mixture prepared in step(6)

8. Effervescent tablets are compressed with suitable punches having average target weight 400 mg/tablet.

### Example5: Granules in Sachet Unit Formula of Eprotirome

**Table 3**

| **Ingredients** | **mg/sachet** | **% (w/w)** |
|---|---|---|
| Eprotirome | 0.2 | 0.05 |
| Sucralose | 65.00 | 17.57 |
| Saccharin | 30.00 | 8.11 |
| Maltodextrin | 224.80 | 60.76 |
| Orange Flavour | 50.00 | 13.51 |
| **Total Tablet Weight** | **370.00** | **100.00** |

### Example 6: Manufacturing Process of Example 5

Pharmaceutical formulation may be prepared using methods as below without limiting scope of this invention;

1. Eprotirome is diluted and mixed geometrically with 10 times fold maltodextrin to obtain homogeneous mixture and sieved with a suitable sieve.

2. Powder mixture prepared in step (1) is mixed geometrically with 10 times fold maltodextrin to obtain a homogenous mixture.

3. Powder mixture in step (2) is mixed with the remaining portion of diluent,sweeteners(sucralose, saccharin and flavour) and sieved with a suitable sieve.

4. Mixture in step (3) is filled into suitable packaging material, such as sachets, having average target powder weight of 370 mg per package.

## Claims

1. An orally disintegrating tablet comprising (i) an effective amount of eprotirome or pharmaceutically acceptable salts thereof and (ii) a pharmaceutically acceptable excipient or mixtures of excipients.

2. According to claim 1, the orally disintegrating tablet dosage form of eprotirome is disintegrated in oral cavity in less than three minutes; more preferably in less than two minutes; most preferably in less than one minute; preferably in less than 30 seconds.

3. The orally disintegrating tablet, as claimed in preceding claims, comprises; eprotirome or pharmaceutically acceptable salt thereof is in the range of from 0.001 % to 50 %, diluent is in the range of from 0.1 % to 99%, binder is in the range of from 0.1 % to 20 %, taste masking agent is in the range of from 0.01 % to 70%, coating agent is in the range of from 0.1 % to 50 %, pore former is in the range of from 0.01 % to 5 %, disintegrant is in the range of from 0.1 % to 60 %, sweetener is in the range of from 0.1 % to 40 %, stabilizing agent is in the range of from 0.1 % to 5 %, glidant is in the range of from 0.1 % to 10 %, lubricant is in the range of from 0.1 % to 10 %, surfactant is in the range of from 0.1 % to 10 %, patient compliance improvizing agents is in the range of from 0.01 % to 50% of total weight of pharmaceutical dosage form **or preferably comprises;** eprotirome or pharmaceutically acceptable salt thereof is in the range of from 0.01 % to 10 %, diluent is in the range of from 10% to 90%, binder is in the range of from 0.1 % to 10 %, taste masking agent is in the range of from 0.1 % to 50%, coating agent is in the range of from 1 % to 20 %, pore former is in the range of from 0.5 % to 3 %, disintegrant is in the range of from 1% to 40 %, sweetener is in the range of from 2 % to 30 %, stabilizing agent is in the range of from 0.5 % to 3 %, glidant is in the range of from 0.2 % to 5 %, lubricant is in the range of from 0.2 % to 5 %, surfactant is in the range of from 1 % to 8 %, patient compliance improvizing agents is in the range of from 0.1% to 30% of total weight of pharmaceutical dosage form or **more preferably comprises**; eprotirome or pharmaceutically acceptable salt thereof is in the range of from 0.1 % to 1 %, diluent is in the range of from 20 % to 70%, binder is in the range of from 1 % to 5 %, taste masking agent is in the range of from 0.2% to 20%, coating agent is in the range of from 2 % to 10 %, pore former is in the range of from 1 % to 2 %, disintegrant is in the range of from 5 % to 20 %, sweetener is in the range of from 5 % to 20 %, stabilizing agent is in the range of from 1 % to 2 %, glidant is in the range of from 0.5 % to 1 %, lubricant is in the range of from 0.5 % to 1 %, surfactant is in the range of from 2 % to 5 %, patient compliance improvizing agents is in the range of from 0.2 % to 20% of total weight of pharmaceutical dosage form.

4. An effervescent tablet comprising (i) an effective amount of eprotirome and (ii) a pharmaceutically acceptable excipient or mixtures of excipients.

5. The effervescent tablet, as claimed in claim 4, is administered as a solution in which drug is suspended or dispersed or dissolved in said solution.

6. The effervescent tablet, as claimed in claims of 4 to 5, comprises; eprotirome or pharmaceutically acceptable salt thereof is in the range of from 0.001 % to 50 %, diluent is in the range of from 0.1 % to 70%, binder is in the range of from 0.1 % to 20 %, taste masking agent is in the range of from 0.01% to 70%, coating agent is in the range of from 0.1 % to 50 %, pore former is in the range of from 0.01 % to 5 %, disintegrant is in the range of from 0.1% to 60 %, sweetener is in the range of from 0.1 % to 40 %, stabilizing agent is in the range of from 0.1 % to 5 %, glidant is in the range of from 0.1 % to 10 %, lubricant is in the range of from 0.1% to 10 %, surfactant is in the range of from 0.1 % to 10 %, patient compliance improvizing agents is in the range of from 0.01% to 50%, acidic and basic effervescent agents are in the range of from 0.1 % to 80%, of total weight of pharmaceutical dosage form. In this formulation or **preferably** comprises; eprotirome or pharmaceutically acceptable salt thereof is in the range of from 0.01 % to 10 %, diluent is in the range of from 10% to 50%, binder is in the range of from 0.1 % to 10 %, taste masking agent is in the range of from 0.1 % to 50%, coating agent is in the range of from 1 % to 20 %, pore former is in the range of from 0.5 % to 3 %, disintegrant is in the range of from 1 % to 40 %, sweetener is in the range of from 2 % to 30 %, stabilizing agent is in the range of from 0.5 % to 3 %, glidant is in the range of from 0.2 % to 5 %, lubricant is in the range of from 0.2 % to 5 %, surfactant is in the range of from 1 % to 8 %, patient compliance improvizing agents is in the range of from 0.1 % to 30%, acidic and basic effervescent agents are in the range of from 1 % to 50%, of total weight of pharmaceutical dosage form **or more preferably comprises;** eprotirome or pharmaceutically acceptable salt thereof is in the range of from 0.1 % to 1 %, diluent is in the range of from 15 % to 30%, binder is in the range of from 1 % to 5 %, taste masking agent is in the range of from 0.2% to 20%, coating agent is in the range of from 2 % to 10 %, pore former is in the range of from 1 % to 2 %, disintegrant is in the range of from 5 % to 20 %, sweetener is in the range of from 5 % to 20 %, stabilizing agent is in the range of from 1 % to 2 %, glidant is in the range of from 0.5 % to 1 %, lubricant is in the range of from 0.5 % to 1 %, surfactant is in the range of from 2 % to 5 %, patient compliance improvizing agents is in the range of from 0.2 % to 20%, acidic and basic effervescent agents are in the range of from 2 % to 20%, of total weight of pharmaceutical dosage form.

7. A unit dose of granules in sachet dosage form comprising (i) an effective amount of eprotirome and (ii) a pharmaceutically acceptable excipient or mixtures of excipients.

8. The sachet formulation, as claimed in claim 7, includes powder, granulate, pellets, spheres, particles or effervescent powder, effervescent granulate, effervescent pellets, effervescent spheres, effervescent particles or mixtures thereof.

9. The sachet, as claimed in claims 7 to 8, comprises; eprotirome or pharmaceutically acceptable salt thereof is in the range of from 0.001 % to 50 %, diluent is in the range of from 1 % to 50%, binder is in the range of from 3 % to 25 %, taste masking agent is in the range of from 1% to 40%, coating agent is in the range of from 0.1 % to 50 %, pore former is in the range of from 0.01 % to 5 %, disintegrant is in the range of from 0.1 % to 60 %, sweetener is in the range of from 0.1 % to 40 %, stabilizing agent is in the range of from 0.1 % to 5 %, glidant is in the range of from 0.1 % to 10 %, lubricant is in the range of from 0.1 % to 10 %, surfactant is in the range of from 0.1 % to 10 %, patient compliance improvizing agents is in the range of from 0.01 % to 50% of total weight of pharmaceutical dosage form or **preferably comprises;** eprotirome or pharmaceutically acceptable salt thereof is in the range of from 0.01 % to 10 %, diluent is in the range of from 10% to 40%, binder is in the range of from 5 % to 20 %, taste masking agent is in the range of from 2% to 20%, coating agent is in the range of from 1 % to 20 %, pore former is in the range of from 0.5 % to 3 %, disintegrant is in the range of from 1% to 40 %, sweetener is in the range of from 2 % to 30 %, stabilizing agent is in the range of from 0.5 % to 3 %, glidant is in the range of from 0.2 % to 5 %, lubricant is in the range of from 0.2 % to 5 %, surfactant is in the range of from 1 % to 8 %, patient compliance improvizing agents is in the range of from 0.1 % to 30% of total weight of pharmaceutical dosage form or **more preferably comprises;** eprotirome or pharmaceutically acceptable salt thereof is in the range of from 0.1 % to 1 %, diluent is in the range of from 18 % to 25%, binder is in the range of from 7 % to 15 %, taste masking agent is in the range of from 5% to 15%, coating agent is in the range of from 2 % to 10 %, pore former is in the range of from 1 % to 2 %, disintegrant is in the range of from 5 % to 20 %, sweetener is in the range of from 5 % to 20 %, stabilizing agent is in the range of from 1 % to 2 %, glidant is in the range of from 0.5 % to 1 %, lubricant is in the range of from 0.5 % to 1 %, surfactant is in the range of from 2 % to 5 %, patient compliance improvizing agents is in the range of from 0.2 % to 20% of total weight of pharmaceutical dosage form.

10. A dry syrup dosage form comprising (i) an effective amount of eprotirome and (ii) a pharmaceutically acceptable excipient or mixtures of excipients.

11. Dry syrup formulation, as claimed in claim 10, comprises dry powder or granules or pellets or mixtures thereof.
